Europäisches Patentamt

European Patent Office

Office européen des brevets

(19)

(11) Veröffentlichungsnummer: **0 291 863 B1**

(12) **EUROPÄISCHE PATENTSCHRIFT**

(45) Veröffentlichungstag der Patentschrift: **22.01.92**

(51) Int. Cl.⁵: **A61K 37/26**

(21) Anmeldenummer: **88107667.3**

(22) Anmeldetag: **13.05.88**

(54) **Mischkristalle aus Insulin und Insulinderivaten, Verfahren zur Herstellung dieser Mischkristalle und diese Mischkristalle enthaltende pharmazeutische Mittel.**

(30) Priorität: **22.05.87 DE 3717370**

(43) Veröffentlichungstag der Anmeldung:
**23.11.88 Patentblatt 88/47**

(45) Bekanntmachung des Hinweises auf die
Patenterteilung:
**22.01.92 Patentblatt 92/04**

(84) Benannte Vertragsstaaten:
**AT BE CH DE ES FR GB GR IT LI LU NL SE**

(56) Entgegenhaltungen:
**EP-A- 0 132 769**
**EP-A- 0 132 770**

(73) Patentinhaber: **HOECHST AKTIENGESELL-
SCHAFT**
**Postfach 80 03 20**
**W-6230 Frankfurt am Main 80(DE)**

(72) Erfinder: **Dörschug, Michael, Dr.**
**Sonnenleite 20**
**W-4630 Bochum 7(DE)**
Erfinder: **Obermeier, Rainer, Dr.**
**Langenhainer Weg 14**
**W-6234 Hattersheim am Main(DE)**

## Beschreibung

Diabetes mellitus ist eine Stoffwechselstörung, die als wesentliches Symptom einen erhöhten Blutzuckerspiegel zeigt. Sie hat ihre Ursache darin, daß das pankreatische Hormon Insulin nicht in genügender Menge freigesetzt wird. Die Substitution des natürlichen Hormons geschieht heute in der Regel durch tierisches Insulin, das aus Schlachttierdrüsen isoliert wird oder humanes Insulin, das semisynthetisch aus Schweineinsulin oder durch gentechnologische Methoden zugänglich ist.

Die spezielle chemische Natur des Insulins bringt es mit sich, daß die Therapie in der Regel parenteral erfolgt; das Hormon würde z.b. bei Magen/Darm-Passage vollständig abgebaut, noch bevor es zur Wirkung kommen kann. Abbaureaktionen, im wesentlichen durch verschiedene, relativ unspezifische proteolytische Enzyme, finden aber auch am Injektionsort und in der Zirkulation statt. Die dadurch bedingte kurze Halbwertszeit in vivo von nur etwa 7 Minuten ist physiologisch im Sinne einer Homöostase im Grunde sinnvoll; die Therapie wird dadurch aber erheblich erschwert, weil der Diabetiker bis zu viermal täglich, in der Regel kurz vor den Mahlzeiten, spritzen muß.

Nun ist die Diabetestherapie gekennzeichnet von individuellen Einflußfaktoren, wie Unterschiede in der Verwertbarkeit der Mahlzeiten, Unterschiede in der Charakteristik des Unterhautgewebes, daneben aber auch die spezifischen Eßgewohnheiten, körperliche Aktivitäten und viele mehr. Es ist somit unabdingbar für eine gute Blutzuckereinstellung, eine Reihe von Insulinpräparaten mit unterschiedlicher Wirkungscharakteristik zur Verfügung zu haben, die den individuellen Bedürfnissen angepaßt sind. Im Zusammenhang mit nicht- optimaler Einstellung werden neben den unmittelbaren subjektiven und objektiven Effekten, wie Hyper- oder Hypoglykämien, insbesondere der Formenkreis der diabetischen Spätschäden, diskutiert. Dazu zählen vor allem Makro- und Mikroangiopathie, Neuropathie, Nephropathie und Retinopathie.

Als Präparationen, die den Bedürfnissen der Patienten angepaßt sind, haben sich neben reinen Verzögerungsinsulinen vor allem sogenannte Intermediärinsuline erwiesen. Sie stellen Mischungen aus einer verzögerten und einer sofort und kurz wirksamen Komponente dar. Solche Mischungen stellen komplizierte Mehrphasensysteme dar, die über lange Zeit hinweg nur in relativ eng begrenten Mischungsverhältnissen stabil bleiben. So ist beispielsweise eine Suspension von 2-Zink-Insulinkristallen vom Schwein nicht frei mischbar mit gelöstem Schweineinsulin. Sofort oder im Lauf der Zeit fällt das zugemischte, gelöste Insulin wegen des relativ hohen Zinkgehalts, der zur Stabilisierung der Kristalle nötig ist, aus. Solche Mischungen sind innerhalb enger Grenzen stabil, wenn als gelöstes Insulin Rinderinsulin (hiermit wird jedoch die Speziesreinheit, eine medizinisch erwünschte Eigenschaft, eingebüßt) oder eine Mischung aus gelöstem Schweineinsulin und des-Phenylalanin(B1)-Schweineinsulin verwendet wird (GB-A-1492837). Vorteilhafter hinsichtlich der Mischbarkeit mit gelöstem Insulin sind Protamin-Insulin-Zubereitungen, wenn als Verzögerungskomponente Kristalle aus Protamin und Insulin im isophanen Verhältnis eingesetzt werden. Mit diesen Präparaten sind NPH (= Neutral-Protamin-Zubereitung nach Hagedorn)-typische Wirkprofile herstellbar. Die Gegenwart des Protamins (als solches oder in Salzform) als Zusatz erscheint zwar vertretbar, weil Protamin ein relativ unbedenkliches körperfremdes Protein ist; trotzdem bleibt es jedoch ein körperfremder Stoff, der zumindest bei - gegen körperfremde Proteine besonders empfindlichen - Patienten zu unerwünschten Reaktionen führen kann.

Wichtig ist deshalb die Bereitstellung stabiler pharmazeutischer Mittel, die eine den individuellen Bedürfnissen des Diabetikers angepaßte wirkungscharakteristik aufweisen und die außerdem nur minimale Mengen körperfremder Hilfsstoffe, insbesondere nur minimale Mengen körperfremder Proteine, oder am besten überhaupt keine solchen Stoffe enthalten. Wirkstoffkombinationen aus nativem Insulin bzw. dessen Des-Phe$^{B1}$-Analogen und Insulin-Derivaten, deren B-Kette C-terminal eine organische Gruppe basischen Charakters trägt, stellen in dieser Hinsicht einen erheblichen Fortschritt dar (vgl. z.B. EP-A-132769).

Insulin-Derivate, die am C-terminalen Ende der B-Kette die Reste Arg-OH oder Arg-Arg-OH tragen, sind bekannt. Diese Derivate entstehen bei der enzymatischen Umwandlung von Proinsulin in Insulin in vivo als natürliche zwischenprodukte und sind auch in kleinen Anteilen in Pankreasextrakten nachweisbar. Die genannten Reste werden normalerweise durch Trypsin und/oder Carboxypeptidase B oder Enzyme mit ähnlicher Spezifität unter Freisetzung des nativen Insulins abgespalten.

Weitere C-terminal basisch modifizierte Insulin-Derivate sind z.B. bekannt aus EP-A-132770 sowie auch EP-A-140084.

Diesen Insulin-Derivaten ist gemeinsam, daß die zusätzliche(n), an der Oberfläche des Moleküls befindliche(n) positive(n) Ladung(en) dem Molekül einen in den Neutralbereich hinein verschobenen isoelektrischen Punkt verleihen. Je nach Derivat werden isoelektrische Punkte von etwa 5,8 bis etwa 8,5, insbesondere etwa 6,2 bis etwa 8,2, in der isoelektrischen Fokussierung gemessen. Damit sind die Derivate im Neutralbereich weniger löslich als natives Insulin, das seinen isoelektrischen Punkt und damit den Bereich maximaler Unlöslichkeit bei pH = etwa 5,4 hat, während es im Neutralbereich normalerweise gelöst vorliegt.

Es kommen therapeutisch interessante Kombinationen zur Anwendung, wie etwa die Mischung von Insulin in gelöster Form oder in Form von NPH-Kristallen oder anderen klassischen Verzögerungsformen + Insulinderivat. Auf diese Weise lassen sich u.a. sehr lang wirksame Präparate mit differenziertem Basalprofil herstellen. Gerade beim Humaninsulin ist dies wünschenswert, da nach den bisherigen Erfahrungen dessen Wirkungsdauer weder in Form der Zinkkristalle noch in Form der NPH-Kristalle ein echtes Ultra-Retard-Profil (wie etwa die analogen Rinderinsulinpräparate) aufweist. Diese bekannten Zubereitungen enthalten Zinkionen von bis zu 1 %, insbesondere aber nicht über 0,8 %, bezogen auf die Insulin-Insulinderivate-Masse. Gewöhnlich genügen zur Kristallisation relativ geringe Zinkmengen von maximal nur etwa 40 $\mu$g/100 internationale Einheiten (I.E.), bevorzugt aber nicht über etwa 30 $\mu$g/100 I.E., die u.U. bereits in der Trockensubstanz enthalten sind. Sie können außerdem Hilfsmittel mit verzögernder Wirkung auf die Insulinfreisetzung, wie Globin oder Protaminsulfat, enthalten.

Neben den Vorteilen hinsichtlich Kristallgröße und deren Homogenität bedingt der relativ geringe Zinkgehalt, der unterhalb der Konzentration liegt, bei der Zink als Depotträger zu betrachten ist, daß die Kristallsuspension frei mit gelöstem Insulin mischbar sind. Es ist somit beispielsweise möglich, Insulinlösungen mit Suspensionen der Insulin-Derivat-Kristalle vor der Applikation zu vereinigen.

Durch Variation der Anteile der einzelnen Komponenten läßt sich das Wirkprofil des so erhaltenen Arzneimittels steuern.

Somit weisen Kristallsuspensionen aus den beschriebenen Derivaten in vorteilhafter Weise jene Eigenschaften auf, die zur Behandlung des Diabetes mellitus wünschenswert sind. Das Verzögerungsprinzip ist den Insulin-Derivaten inhärent und geht auf ein proteinchemisches Phänomen, die Schwerlöslichkeit am isoelektrischen Punkt, zurück. Es ergibt in der Praxis ein echtes Ultra-Retard-Profil.

Darüberhinaus ist es aber auch wünschenswert, neben dem Ultra-Retard-Profil mittlere Retard-Profile zur Verfügung zu haben.

Diese Aufgabe konnte erfindungsgemäß durch die Erzeugung und ellung neuer Mischkristalle aus

A. nativem Insulin, des-Phe-B1-Insulin, des-Thr-B30-(Human)-oder des-Ala-B30-(Rind, Schwein)-Insulin
und

B. mindestens einem am C-terminalen Ende der B-Kette basisch modifizierten Insulin

gelöst werden.

Die für die Komponente A in Frage kommenden Stoffe sind solche mit einem isoelektrischen Punkt von kleiner oder gleich etwa 5,5; die basisch modifizierten Insuline der Komponente B sind solche mit einem isoelektrischen Punkt zwischen etwa 5,8 und etwa 8,5.

Die erfindungsgemäßen Mischkristalle zeigen gegenüber den bekannten rein physikalischen Gemischen der gleichen Einzelkomponenten trotz ihrer vergleichbaren Stabilität (ohne oder bei nur niedrigem Zinkgehalt) eine deutliche Verringerung der extrem langen Wirkdauer mit einer Wirkstärke, wie sie mit klassischen Depot-Insulin-Präparaten (mit Protamin-Insulin-Kristallen oder 2-Zinkinsulin-Kristallen) - deren Wirkdauer weniger lang ist - zur Behandlung von Diabetes mellitus Verwendung finden. Dabei ist jedoch die Wirkdauer gegenüber den klassischen Depot-Insulin-Präparaten deutlich verlängert; sie liegt also etwa zwischen der Wirkdauer der genannten klassischen Depot-Insulinpräparate und der extrem langen Wirkdauer der rein physikalischen Gemische der Einzelkomponenten A und B. Dieser Effekt ist besonders im Falle der Humaninsulin-Therapie wünschenswert und war bisher nicht möglich. Es ist außerordentlich überraschend, daß die erfindungsgemäßen Mischkristalle gerade diesen Effekt zeigen.

Der notwendige rasche Wirkungseintritt des Insulinanteiles A geht trotz der Mischkristallisation nicht verloren und kann gegebenenfalls durch mechanische bzw. physikalische Mischung mit gelöstem Insulin beschleunigt werden.

Der unerwartete, therapeutisch außerordentlich nützliche Effekt kommt wahrscheinlich durch die leichtere Auflösbarkeit des Kristallgitterverbandes der basisch modifizierten Insuline B bei physiologischen pH-Werten nach gezieltem Einbau von Störstellen mit Hilfe der Komponente A mit niedrigerem isoelektrischen Punkt zustande.

Das bevorzugte Gewichtsverhältnis der Komponenten A und B in den erfindungsgemäßen Mischkristallen liegt bei etwa (10-90):(90-10). Dies bedeutet, daß jede der beiden Komponenten A und B normalerweise zu mindestens etwa 10 Gew.-% in den Mischkristallen vorliegen soll.

Mischkristallkomponente A ist natives Insulin - hauptsächlich Human-, Schweine- oder Rinderinsulin -, des-Phe-B1-Insulin (vorzugsweise Human, Schwein oder Rind) und des-Thr-B30-Humaninsulin oder des-Ala-B30(Schwein,Rind)-Insulin. Im allgemeinen besteht die Komponente A nur aus einem Vertreter dieser Insuline, wobei (natives) Humaninsulin bevorzugt ist.

Die Mischkristallkomponente B wird von mindestens einem am C-terminalen Ende der B-Kette basisch modifizierten Insulin gebildet. Als solche entsprechend basisch modifizierten Insuline kommen z.B. die Insulin-Derivate gemäß Formel I in EP-A-132769, EP-A-132770 und EP-A-140084 in Frage. Bevorzugte Komponenten B sind Arg-B31-

Humaninsulin und Arg$_2$-(B31-32)-Humaninsulin.

Die Komponente B kann sowohl aus einer als auch aus mehreren Einzelverbindungen bestehen. Die Einzelverbindungen wie z.B. die beiden zuletzt genannten Humaninsulin-Derivate, können praktisch in jeder beliebigen Mischung vorhanden sein.

Die Komponenten A und B sind weiterhin in den Mischkristallen vorzugsweise von der gleichen Spezies (also z.B. beides Human oder Schwein).

Besonders bevorzugte Mischkristalle enthalten als Komponente A Humaninsulin und als Komponente B Arg-B31-Humaninsulin oder Arg$_2$-(B31-32)-Humaninsulin oder eine beliebige Mischung von letzteren.

Die Mischkristalle aus den Komponenten A und B können erfindungsgemäß nur aus einer Lösung eines ziemlich engen pH-Bereichs erhalten werden; außerhalb dieses engen pH-Bereiches werden überraschenderweise keine - oder jedenfalls keine optimalen - Mischkristalle mehr erhalten.

Das erfindungsgemäße Verfahren zur Herstellung dieser Mischkristalle ist dadurch gekennzeichnet, daß man eine wäßrige Lösung aus

    A. nativem Insulin, des-Phe-B1-Insulin, des-Thr-B30-(Human)-oder des-Ala-B30-(Schwein,Rind)-Insulin,

    B. mindestens einem am C-terminalen Ende der B-Kette basisch modifizierten Insulin,

mindestens einem physiologisch verträglichen Konservierungsmittel,

mindestens einem physiologisch verträglichen Isotoniemittel,

mindestens einer physiologisch verträglichen Säure,

sowie gegebenenfalls weiteren physiologisch verträglichen Zusatz- und Hilfsstoffen,

des pH-Wertes von etwa 2,5 bis etwa 3,5 herstellt, daß man die Lösung dann durch Zusatz einer physiologisch verträglichen Base sowie gegebenenfalls eines physiologisch verträglichen Puffers auf einen pH-Wert von etwa 5,5 bis etwa 6,9, vorzugsweise von etwa 5,9 bis etwa 6,5, und die Mischkristalle aus den Komponenten A und B aus dieser Lösung zur Kristallisation bringt.

Die Gesamtkonzentration der Komponenten A und B in der wäßrigen Lösung vor der Kristallisation kann in einem relativ weiten Bereich schwanken; bevorzugt ist jedoch eine Konzentration zwischen etwa 0,2 und etwa 40 mg/ml, insbesondere von etwa 1 bis etwa 7,5 mg/ml.

Als Komponenten A werden bevorzugt solche mit einem isoelektrischen Punkt von kleiner oder gleich etwa 5,5 eingesetzt; die in Frage kommenden Komponenten B sollen einen isoelektrischen Punkt zwischen etwa 5,8 und 8,5 besitzen.

Ansonsten gilt für die Komponenten A und B das Gleiche wie bereits vorstehend bei der Beschreibung der erfindungsgemäßen Mischkristalle ausgeführt. Bevorzugte Komponente A ist Humaninsulin, bevorzugte Komponente B Arg-B31-Humaninsulin und/oder Arg$_2$-(B31-32)-Humaninsulin. Im Falle des Einsatzes von mehr als einem Vertreter der Komponente B kann durch deren Mischungsverhältnis das gewünschte Blutzuckersenkungsprofil gut eingestellt werden.

Die Komponenten A und B können ihrerseits im Prinzip in beliebigem Mischungsverhältnis werden; bevorzugt ist jedoch ein Gewichtsverhältnis von etwa (10-90):(90-10). Etwa in dem gleichen Gewichtsverhältnis, welches in der Ausgangslösung eingestellt wird, finden sich die Komponenten A und B dann auch in den Mischkristallen wieder.

Als physiologisch verträgliche Konservierungsmittel können die für solche Zwecke üblichen und bekannten Mittel verwendet werden, also z.B. aromatische Hydroxyverbindungen wie Phenol, m-Cresol und/oder p-Hydroxybenzoesäureester (von letzterem hauptsächlich der Ethylester). Auch die Konzentration des bzw. der Konservierungsmittel soll im üblichen Rahmen liegen. Zweckmäßige Konzentrationen liegen zwischen etwa 0,02 und etwa 1 (Gew.)%.

Als physiologisch verträgliche Isotoniemittel kommen ebenfalls die für solche Zwecke üblichen Verbindungen in Frage wie z.B. Glycerin und/oder NaCl. Auch deren Konzentration soll im üblichen Rahmen liegen; d.h. hier also vorteilhaft bei etwa 300 Milli-Osmol.

Als physiologisch verträgliche Säuren (zur Einstellung des pH-Wertes) werden z.B. Essigsäure, Zitronensäure oder Phosphorsäure eingesetzt. Ihre Konzentration ergibt sich im wesentlichen durch die pH-Werte-Begrenzung der Lösung.

In Frage kommende physiologisch verträgliche Basen sind z.B. NaOH oder, KOH. und physiologisch verträgliche Puffer z.B. Natriumacetat, -citrat, -phosphat oder Tris-(hydroxymethyl-)aminomethan.

Die Kristallisationslösung kann gegebenenfalls natürlich auch weitere physiologisch verträgliche Zusatz- und Hilfsstoffe wie z.B. ein Zn-Salz, enthalten.

Nach dem Einstellen des pH-Wertes der (zunächst ziemlich sauren) Kristallisationslösung auf etwa 5,5 bis etwa 6,9, vorzugsweise von etwa 5,9 bis etwa 6,5, läßt man die Lösung bei einer Temperatur von vorzugsweise etwa 3 bis 27°C, insbesondere von etwa 10 bis 20°C, stehen; die Mischkristalle aus A und B kristallisieren dann mehr oder weniger schnell aus.

Sie können zusammen mit der überstehenden Lösung gleich als entsprechende pharmazeutische Mittel verwendet werden. Wenn der Lösung Zn-Ionen (in Form entsprechender Verbindungen) als Depot-Hilfsmittel zugesetzt wurden, soll die Menge vorzugsweise so eingestellt worden sein, daß die resultierende Mischkristallsuspension bis zu etwa 100 μg/100 I.E. Zn-Ionen enthält. Als Variante dieser Zubereitung können die so erzeugten Mischkristalle auch durch Abschleudern isoliert werden und nach Gefriertrocknung in einem Placebo-Puffer dosisgemäß resuspendiert werden.

Die erfindungsgemäßen Mischkristalle sowie die entsprechenden pharmazeutischen Zubereitungen, welche diese Mischkristalle enthalten, eignen sich wegen des mittleren Retard-Profils und der Möglichkeit der "Feineinstellung" durch Variation der Art und Menge insbesondere der Einzelverbindungen der Komponente B hervorragend zur Behandlung von Diabetes mellitus.

Die Erfindung wird nun durch die folgenden Beispiele näher erläutert.

## Beispiel 1

Mischkristallsuspension aus 25 % Humaninsulin, 75 % $Arg_2$-(B31-32)-humaninsulin mit einer Gesamtaktivität von 40 I.E./ml.

Man löst in einem Gesamtvolumen von 100 ml $H_2O$

| | |
|---|---|
| $Arg_2$-(B31-32)-humaninsulin (27 I.E./mg)- | 111 mg |
| Humaninsulin (27 I.E./mg) | 37 mg |
| Citronensäure-1-hydrat | 1050 mg |
| Glycerin | 1600 mg |
| Phenol | 65 mg |
| m-Cresol | 165 mg |

Der pH-Wert der Lösung liegt bei ca. 3,5. Die Lösung wird mit 1N NaOH zur Kristallisation auf pH 6,3 eingestellt. Nach vollständiger Kristallisation über Nacht bei Raumtemperatur lassen sich im Überstand mit Hilfe der HPLC ( = High pressure liquid chromatography) 3-5 % Humaninsulin/$Arg_2$-(B31-32)-humaninsulin nachweisen. Ihr Verhältnis beträgt wie bei der Kokristallisation 25/75 %; bei s.c.( = subcutan)-Application am Kaninchen von 100 μl des klaren Überstandes läßt sich keine signifikante Blutzuckersenkung messen (weil die Lösung praktisch keinen Wirkstoff mehr enthält).

## Beispiel 2

Wie in Beispiel 1 werden mischkristallisiert:

| | |
|---|---|
| $Arg_2$-(B31-32)-humaninsulin | 74 mg |
| Arg-B31-humaninsulin (27 I.E./mg) | 37 mg |
| Humaninsulin | 37 mg |

Das Kristall-Sediment wird abgeschleudert, mit Puffer gewaschen und gefriergetrocknet. Das gefriergetrocknete Kokristall-Pulver wird mit 40 I.E./ml im Placebo-Puffer von pH 6,5 aufgeschlämmt.

## Beispiel 3

| | |
|---|---|
| Humaninsulin (27 I.E./mg) | 37 mg |
| Arg-B31-humaninsulin (27 I.E./mg) | 111 mg |

werden wie in Beispiel 2 mischkristallisiert, isoliert und gefriergetrocknet. Eine Aufschlämmung des Kristallpulvers in Placebo-Puffer von pH 6,3 (40 I.E./ml) zeigt mit 0,2 I.E./kg s.c. appliziert, am Hund eine deutlich verzögerte Blutzuckersenkung, die mit der von NPH-Insulin vergleichbar ist.

## Patentansprüche

## Patentansprüche für folgende Vertragsstaaten : AT, BE, CH, DE, FR, GB, IT, LI, LU, NL, SE

1. Mischkristalle aus
   A. nativem Insulin, des-Phe-B1-insulin, des-Thr-B30 (Human)- oder des-Ala-B30-(Schwein, Rind)-insulin
   und
   B. mindestens einem am C-terminalen Ende der B-Kette basisch modifizierten Insulin.

2. Mischkristalle nach Anspruch 1, gekennzeichnet durch einen isoelektrischen Punkt der Komponente A kleiner oder gleich etwa 5,5 und der Komponente B bei etwa 5,8 bis etwa 8,5.

3. Mischkristalle nach Anspruch 1 oder 2, gekennzeichnet durch ein Gewichtsverhältnis der beiden Komponenten A und B von etwa (10-90):(90-10).

4. Mischkristalle nach einem oder mehreren der Ansprüche 1 - 3, dadurch gekennzeichnet, daß die Komponente A Humaninsulin und die Komponente B Arg-B31-Humaninsulin und/oder $Arg_2$-(B31-32)-Humaninsulin ist.

5. Verfahren zur Herstellung von Mischkristallen gemäß der Definition in einem oder mehreren der Ansprüche 1 - 4, dadurch gekennzeichnet, daß man eine wäßrige Lösung von
   A. nativem Insulin, des-Phe-B1-insulin, des-Thr-B30 (Human)- oder des-Ala-B30-(Schwein, Rind)-insulin und
   B. mindestens einem am C-terminalen Ende der B-Kette basisch modifizierten Insulin,
   mindestens einem physiologisch verträglichen Konservierungsmittel,
   mindestens einem physiologisch verträglichen Isotoniemittel,
   mindestens einer physiologisch verträglichen Säure,
   sowie gegebenenfalls weiteren physiologisch verträglichen Zusatz- und Hilfsstoffen
   des pH-Werts von etwa 2,5 bis etwa 3,5 herstellt,

daß man die Lösung dann durch Zusatz einer physiologisch verträglichen Base sowie gegebenenfalls eines physiologish verträglichen Puffers auf einen pH-Wert von etwa 5,5 bis 6,9, vorzugsweise von etwa 5,9 bis etwa 6,5, und die Mischkristalle aus den Komponenten A und B aus dieser Lösung zur Kristallisation bringt.

6. Verfahren nach Anspruch 5, dadurch gekennzeichnet, daß man die Gesamtkonzentration der Komponenten A und B in der wäßrigen Lösung vor der Kristallisation auf etwa 0,2 bis etwa 40 mg/ml, insbesondere auf etwa 1 bis etwa 7,5 mg/ml, einstellt.

7. Verfahren nach Anspruch 5 oder 6, dadurch gekennzeichnet, daß man als Komponente A eine solche mit dem isoelektrischen Punkt kleiner oder gleich 5,5 - vorzugsweise Humaninsulin - und als Komponente B mindestens eine solche mit einem isoelektrischen Punkt zwischen etwa 5,8 und etwa 8,5 - vorzugsweise Arg-B31-Humaninsulin und/oder $Arg_2$-(B31-32)-Humaninsulin - verwendet.

8. Verfahren nach einem odere mehreren der Ansprüche 5 - 7, dadurch gekennzeichnet, daß man die Komponenten A und B im Gewichtsverhältnis von etwa (10-90):(90-10) einsetzt.

9. Verfahren nach einem oder mehreren der Ansprüche 5 - 8, dadurch gekennzeichnet, daß man als physiologisch verträgliche Konservierungsmittel Phenol, m-Cresol und/oder p-Hydroxybenzoesäurethylester, als physiologisch verträgliche Isotoniemittel Glycerin und/oder NaCl, als physiologisch verträgliche Säuren Essigsäure, Zitronensäure und/oder Phosphorsäure, als physiologisch verträgliche Basen NaOH und/oder KOH und als physiologisch verträgliche Puffer Natriumacetat, -citrat und/oder -phosphat und/oder Tris-(hydroxymethyl-) aminomethan verwendet.

10. Pharmazeutische Mittel, enthaltend die Mischkristalle gemäß einem oder mehreren der Ansprüche 1 - 4 oder wie erhalten nach einem oder mehreren der Ansprüche 5 - 9 in einem physiologisch verträglichen Träger.

11. Pharmazeutische Mittel gemäß Anspruch 10 zur Behandlung von Diabetes mellitus.

12. Verwendung der Mischkristalle gemäß einem oder mehreren der Ansprüche 1 - 4 oder wie erhalten nach einem oder mehreren der Ansprüche 5 -9 sowie des pharmazeutischen Mittels gemäß Anspruch 10 zur Herstellung eines Arzneimittels zur Behandlung von Diabetes mellitus.

**Patentansprüche für folgende Vertragsstaaten : ES, GR**

1. Verfahren zur Herstellung von Mischkristallen aus

    A. Nativem Insulin, des-Phe-B1-insulin, des-Thr-B30 (Human)- oder des-Ala-B30-(Schwein, Rind)-insulin und
    B. mindestens einem am C-terminalen Ende der B-Kette basisch modifizierten Insulin,

    dadurch gekennzeichnet,
    daß man eine wäßrige Lösung von
    A. nativem Insulin, des-Phe-B1-insulin, des-Thr-B30 (Human)- oder des-Ala-B30-(Schwein, Rind)-insulin und
    B. mindestens einem am C-terminalen Ende der B-Kette basisch modifizierten Insulin,
    mindestens einem physiologisch verträglichen Konservierungsmittel,
    mindestens einem physiologisch verträglichen Isotoniemittel,

    mindestens einer physiologisch verträglichen Säure,
    sowie gegebenenfalls weiteren physiologisch verträglichen Zusatz- und Hilfsstoffen
    des pH-Werts von etwa 2,5 bis etwa 3,5 herstellt,
    daß man die Lösung dann durch Zusatz einer physiologisch verträglichen Base
    sowie gegebenenfalls eines physiologisch verträglichen Puffers
    auf einen pH-Wert von etwa 5,5 bis 6,9, vorzugsweise von etwa 5,9 bis etwa 6,5,
    und die Mischkristalle aus den Komponenten A und B aus dieser Lösung zur Kristallisation bringt.

2. Verfahren nach Anspruch 1, dadurch gekennzeichnet, daß man als Komponente A eine solche mit einem isoelektrischen Punkt kleiner oder gleich etwa 5,5 und als Komponente B eine solche mit einem isoelektrischen Punkt zwischen etwa 5,8 bis etwa 8,5 verwendet.

3. Verfahren nach Anspruch 1 oder 2, dadurch gekennzeichnet, daß man die Komponenten A und B in einem Gewichtsverhältnis von etwa (10-90):(90-10) einsetzt.

4. Verfahren nach einem oder mehreren der Ansprüche 1-3, dadurch gekennzeichnet, daß man als Komponente A Humaninsulin und als Komponente B Arg-B31-Humaninsulin und/oder $Arg_2$-(B31-32)-Humaninsulin verwendet.

5. Verfahren nach einem oder mehreren der Ansprüche 1-4, dadurch gekennzeichnet, daß man die Gesamtkonzentration der Komponenten A und B in der wäßrigen Lösung vor der Kristallisation auf etwa 0,2 bis etwa 40 mg/ml, insbesondere auf etwa 1 bis etwa 7,5 mg/ml, einstellt.

6. Verfahren nach einem oder mehreren der Ansprüche 1-5, dadurch gekennzeichnet, daß man als
physiologisch verträgliche Konservierungsmittel Phenol, m-Cresol und/oder p-Hydroxybenzoesäurethylester,
als physiologisch verträgliche Isotoniemittel Glycerin und/oder NaCl,
als physiologisch verträgliche Säuren Essigsäure, Zitronensäure und/oder Phosphorsäure,
als physiologisch verträgliche Basen NaOH und/oder KOH und
als physiologisch verträgliche Puffer Natriumacetat, -citrat und/oder -phosphat und/oder Tris-(hydroxymethyl-) aminomethan verwendet.

7. Verfahren zur Herstellung eines pharmazeutischen Mittels, dadurch gekennzeichnet, daß man Mischkristalle, welche nach dem Verfahren gemäß einem oder mehreren der Ansprüche 1 - 6 erhalten wurden, mit einem physiologisch verträglichen Träger sowie ggf. weiteren physiologisch verträglichen Zusatz- und Hilfsstoffen in eine geeignete Darreichungsform bringt.

8. Verfahren nach Anspruch 7, dadurch gekennzeichnet, daß das verfahrensgemäß hergestellte Mittel zur Behandlung von Diabetes mellitus bestimmt ist.

9. Verwendung der nach dem Verfahren gemäß einem oder mehreren der Ansprüche 1 - 6 erhaltenen Mischkristalle zur Herstellung eines pharmazeutischen Mittels zur Behandlung von Diabetes mellitus.

**Claims**
**Claims for the following Contracting States :**
**AT, BE, CH, DE, FR, GB, IT, LI, LU, NL, SE**

1. Mixed crystals composed of
   A. unmodified insulin, des-Phe-B1-insulin, des-Thr-B30-(human)- or des-Ala-B30(pork, beef)-insulin and
   B. at least one insulin having a basic modification at the C-terminal end of the B chain.

2. Mixed crystals as claimed in claim 1, in which the isoelectric point of component A is less than or equal to about 5.5, and that of component B is about 5.8 to about 8.5.

3. Mixed crystals as claimed in claim 1 or 2, wherein the ratio by weight of the two components A and B is about (10-90):(90-10).

4. Mixed crystals as claimed in one or more of claims 1-3, wherein component A is human insulin, and component B is Arg-B31-human insulin and/or $Arg_2$-(B31-32)-human insulin.

5. A process for the preparation of mixed crystals as defined in one or more of claims 1-4, which comprises preparing an agueous solution composed of
   A. unmodified insulin, des-Phe-Bl-insulin, des-Thr-B30-(human)- or des-Ala-B30(pork, beef)-insulin and
   B. at least one insulin having a basic modification at the C-terminal end of the B chain,
   at least one physiologically tolerated preservative, at least one physiologically tolerated tonicity agent, at least one physiologically tolerated acid,
   and, where appropriate, other physiologically tolerated additives and auxiliaries,
   of pH from about 2.5 to about 3.5,
   and comprises the solution then being adjusted by addition of a physiologically tolerated base and, where appropriate,
   of a physiologically tolerated buffer;
   to a pH of from about 5.5 to 6.9, preferably of from about 5.9 to about 6.5,
   and inducing the crystallization of the mixed crystals composed of components A and B from this solution.

6. The process as claimed in claim 5, wherein the total concentration of components A and B in the aqueous solution before the crystallization is adjusted to about 0.2 to about 40 mg/ml, in particular to about 1 to about 7.5 mg/ml.

7. The process as claimed in claim 5 or 6, wherein the component A which is used is one having an isoelectric point which is less than or equal to 5.5 - preferably human insulin - and the component B which is used is at least one which has an isoelectric point between about 5.8 and about 8.5 - preferably Arg-B31-human insulin and/or $Arg_2$-(B31-32)- human insulin.

8. The process as claimed in one or more of claims 5 - 7, wherein components A and B are used in the ratio by weight of about (10-90):-(90-10).

9. The process as claimed in one or more of claims 5 - 8, wherein the physiologically tolerated preservatives which are used are phenol, m-cresol and/or ethyl p-hydroxybenzoate, the physiologically tolerated tonicity agents which are used are glycerol and/or NaCl, the physiologically tolerated acids which are used are acetic acid, citric acid and/or phosphoric acid, the physiologically tolerated bases which are used are NaOH and/or KOH, and the physiologically tolerated buffers which are used are sodium acetate, citrate and/or phosphate, and/or tris(hydroxymethyl)aminomethane

10. A pharmaceutical agent containing the mixed crystals as claimed in one or more of claims 1 - 4, or as obtained as claimed in one or more of claims 5 - 9, in a physiologically tolerated vehicle.

11. A pharmaceutical agent as claimed in claim 10 for the treatment of diabetes mellitus.

12. The use of the mixed crystals as claimed in one or more of claims 1 - 4, or as obtained as claimed in one or more of claims 5 - 9, and of the pharmaceutical agent as claimed in claim 10, for the preparation of a medicament for the treatment of diabetes mellitus.

**Claims for the following Contracting States : ES, GR**

1. A process for the preparation of mixed crystals composed of
A. unmodified insulin, des-Phe-B1-insulin, des-Thr-B30-(human)- or des-Ala-B30(pork, beef)-insulin and
B. at least one insulin having a basic modification at the C-terminal end of the B chain which comprises preparing an agueous solution composed of
A. unmodified insulin, des-Phe-Bl-insulin, des-Thr-B30-(human)- or des-Ala-B30(pork, beef)-insulin and
B. at least one insulin having a basic modification at the C-terminal end of the B chain, at least one physiologically tolerated preservative, at least one physiologically tolerated tonicity agent,
at least one physiologically tolerated acid,
and, where appropriate, other physiologically tolerated additives and auxiliaries,
of pH from about 2.5 to about 3.5,
and comprises the solution then being adjusted by addition of a physiologically tolerated base and, where appropriate, of a physiologically tolerated buffer,
to a pH of from about 5.5 to 6.9, preferably of from about 5.9 to about 6.5,
and inducing the crystallization of the mixed crystals composed of components A and B from this solution.

2. The process as claimed in claim 1, wherein the component A used is one with an isoelectric point less than or equal to about 5.5 and the component B used is one with an isoelectric point between about 5.8 and about 8.5.

3. The process as claimed in claim 1 or 2, wherein the two components A and B are used in the ratio by weight of about (10-90):(90-10).

4. The process as claimed in one or more of claims 1 - 3, wherein the component A used is human insulin, and the component B used is Arg-B31-human insulin and/or $Arg_2$-(B31-32)-human insulin.

5. The process as claimed in one or more of claims 1 - 4, wherein the total concentration of components A and B in the agueous solution before the crystallization is adjusted to about 0.2 to about 40 mg/ml, in particular to about 1 to about 7.5 mg/ml.

6. The process as claimed in one or more of claims 1 - 5, wherein the physiologically tolerated preservatives which are used are phenol, m-cresol and/or ethyl p-hydroxybenzoate, the physiologically tolerated tonicity agents which are used are glycerol and/or NaCl, the physiologically tolerated acids which are used are acetic acid, citric acid and/or phosphoric acid, the physiologically tolerated bases which are used are NaOH and/or KOH, and the physiologically tolerated buffers which are used are sodium acetate, citrate and/or phosphate, and/or tris(hydroxymethyl)aminomethane.

7. A process for the preparation of a pharmaceutical agent, which comprises converting mixed crystals which have been obtained by the process as claimed in one or more of claims 1 - 6 with a physiologically tolerated vehicle and, where appropriate, other physiologically tolerated additives and auxiliaries into a suitable dosage form.

8. The process as claimed in claim 7, wherein the agent prepared by the process is intended for the treatment of diabetes mellitus.

9. The use of the mixed crystals obtained by the process as claimed in one or more of claims 1 - 6 for the preparation of a pharmaceutical agent for the treatment of diabetes mellitus.

**Revendications**
**Revendications pour les Etats contractants suivants : AT, BE, CH, DE, FR, GB, IT, LI, LU, NL, SE**

1. Cristaux mixtes de
   A. insuline native, des-Phe-B1-insuline, des-Thr-B30-insuline humaine ou des-Ala-B30-insuline (porcine, bovine), et
   B. au moins une insuline à modification basique à l'extrémité C-terminale de la chaîne B.

2. Cristaux mixtes selon la revendication 1, caractérisés par un point isoélectrique du composant A ≤ 5,5 environ et un point isoélectrique du composant B d'environ 5,8 à environ 8,5.

3. Cristaux mixtes selon la revendication 1 ou 2, caractérisés par un rapport pondéral des deux composants A et B allant d'environ 10:90 à 90:10.

4. Cristaux mixtes selon une ou plusieurs des revendications 1 à 3, caractérisés en ce que le composant A est l'insuline humaine et le composant B est l'Arg-B31-insuline humaine et/ou l'Arg$_2$-(B31-32)-insuline humaine.

5. Procédé pour la préparation de cristaux mixtes selon la définition dans une ou plusieurs des revendications 1 à 4, caractérisé en ce que l'on prépare une solution aqueuse à partir de
   A. insuline native, des-Phe-B1-insuline, des-Thr-B30-insuline (humaine) ou des-Ala-B30-insuline (porcine, bovine),
   B. au moins une insuline à modification basique à l'extrémité C-terminale de la chaîne B,
   au moins un conservateur physiologiquement acceptable,
   au moins un agent d'isotonie physiologiquement acceptable,
   au moins un acide physiologiquement acceptable,
   ainsi qu'éventuellement d'autres additifs et adjuvants physiologiquement acceptables,
ayant un pH d'environ 2,5 à environ 3,5,
on porte ensuite la solution à un pH d'environ 5,5 à environ 6,9, de préférence d'environ 5,9 à environ 6,5, par addition d'une base physiologiquement acceptable ainsi qu'éventuellement d'un tampon physiologiquement acceptable,
et on fait cristalliser à partir de cette solution les cristaux mixtes constitués des composants A et B.

6. Procédé selon la revendication 5, caractérisé en ce l'on ajuste la concentration totale des composants A et B dans la solution aqueuse, avant la cristallisation, aux environs de 0,2 à 40 mg/ml, en particulier aux environs de 1 à 7,5 mg/ml.

7. Procédé selon la revendication 5 ou 6, caractérisé en ce l'on utilise, en tant que composant A, un tel composant ayant un point isoélectrique ≤ 5,5 - de préférence l'insuline humaine - et, en tant que composant B, au moins un tel composant ayant un point isoélectrique compris entre environ 5,8 et environ 8,5, de préférence l'Arg-B31-insuline humaine et/ou l'Arg$_2$-(B31-32)-insuline humaine.

8. Procédé selon une ou plusieurs des revendications 5 à 7, caractérisé en ce que l'on utilise les composants A et B en un rapport pondéral allant d'environ de 10:90 à 90:10.

9. Procédé selon une ou plusieurs des revendications 5 à 8, caractérisé en ce que l'on utilise en tant que conservateurs physiologiquement acceptables, le phénol, le m-crésol et/ou le p-hydroxybenzoate d'éthyle,
en tant qu'agents d'isotonie physiologiquement acceptables, le glycérol et/ou NaCl,
en tant qu'acides physiologiquement acceptables, l'acide acétique, l'acide citrique et/ou l'acide phosphorique,
en tant que bases physiologiquement acceptables, NaOH et/ou KOH, et
en tant que tampons physiologiquement acceptables, l'acétate, le citrate, et/ou le phosphate de sodium et/ou le tris(hydroxyméthyl)-aminométhane.

10. Produit pharmaceutique contenant les cristaux mixtes selon une ou plusieurs des revendications 1 à 4, ou tels qu'obtenus selon une ou plusieurs des revendications 5 à 9, dans un véhicule physiologiquement acceptable.

11. Produit pharmaceutique selon la revendication 10, pour le traitement du diabète sucré.

**12.** Utilisation des cristaux mixtes selon une ou plusieurs des revendications 1 à 4, ou tels qu'obtenus selon une ou plusieurs des revendications 5 à 9, ainsi que du produit pharmaceutique selon la revendication 10, pour la fabrication d'un médicament pour le traitement du diabète sucré.

**Revendications pour les Etats contractants suivants : ES, GR**

**1.** Procédé pour la préparation de cristaux mixtes de

A. insuline native, des-Phe-B1-insuline, des-Thr-B30-insuline (humaine) ou des-Ala-B30-insuline (porcine, bovine), et

B. au moins une insuline à modification basique à l'extrémité C-terminale de la chaîne B,

caractérisé en ce que l'on prépare une solution aqueuse à partir de

A. insuline native, des-Phe-B1-insuline, des-Thr-B30-insuline (humaine) ou des-Ala-B30-insuline (porcine, bovine),

B. au moins une insuline à modification basique à l'extrémité C-terminale de la chaîne B,

au moins un conservateur physiologiquement acceptable,

au moins un agent d'isotonie physiologiquement acceptable,

au moins un acide physiologiquement acceptable,

ainsi qu'éventuellement d'autres additifs et adjuvants physiologiquement acceptables,

ayant un pH d'environ 2,5 à environ 3,5,

on porte ensuite la solution à un pH d'environ 5,5 à environ 6,9, de préférence d'environ 5,9 à environ 6,5, par addition d'une base physiologiquement acceptable ainsi qu'éventuellement d'un tampon physiologiquement acceptable,

et on fait cristalliser à partir de cette solution les cristaux mixtes constitués des composants A et B.

**2.** Procédé selon la revendication 1, caractérisé en ce que l'on utilise en tant que composant A un tel composant ayant un point isoélectrique ≤ 5,5, et, en tant que composant B, un tel composant ayant un point isoélectrique compris entre environ 5,8 et environ 8,5.

**3.** Procédé selon la revendication 1 ou 2, caractérisé en ce que l'on utilise les composants A et B en un rapport pondéral allant d'environ 10:90 à 90:10.

**4.** Procédé selon une ou plusieurs des revendications 1 à 3, caractérisé en ce que l'on utilise en tant que composant A l'insuline humaine, et, en tant que composant B, l'Arg-B31-insuline humaine et/ou l'Arg$_2$-(B31-32)-insuline humaine.

**5.** Procédé selon une ou plusieurs des revendications 1 à 4, caractérisé en ce que l'on ajuste la concentration totale des composants A et B dans la solution aqueuse, avant la cristallisation, aux environs de 0,2 à 40 mg/ml, en particulier aux environs de 1 à 7,5 mg/ml.

**6.** Procédé selon une ou plusieurs des revendications 1 à 5, caractérisé en ce que l'on utilise

en tant que conservateurs physiologiquement acceptables, le phénol, le m-crésol et/ou le p-hydroxybenzoate d'éthyle,

en tant qu'agents d'isotonie physiologiquement acceptables, le glycérol et/ou NaCl,

en tant qu'acides physiologiquement acceptables, l'acide acétique, l'acide citrique et/ou l'acide phosphorique,

en tant que bases physiologiquement acceptables, NaOH et/ou KOH, et

en tant que tampons physiologiquement acceptables, l'acétate, le citrate, et/ou le phosphate de sodium et/ou le tris(hydroxyméthyl)-aminométhane.

**7.** Procédé pour la préparation d'un produit pharmaceutique, caractérisé en ce que l'on met sous une forme pharmaceutique appropriée des cristaux mixtes, qui ont été obtenus par le procédé selon une ou plusieurs des revendications 1 à 6, avec un véhicule physiologiquement acceptable ainsi qu'éventuellement d'autres additifs et adjuvants physiologiquement acceptables.

**8.** Procédé selon la revendication 7, caractérisé en ce que le produit préparé selon l'invention est destiné au traitement du diabète sucré.

**9.** Utilisation des cristaux mixtes obtenus par le procédé selon une ou plusieurs des revendications 1 à 6, pour la fabrication d'un produit pharmaceutique pour le traitement du diabète sucré.